(19) Europäisches Patentamt
European Patent Office
Office européen des brevets

(11) Veröffentlichungsnummer: **0 037 584**
**B1**

(12) # EUROPÄISCHE PATENTSCHRIFT

(45) Veröffentlichungstag der Patentschrift: **16.11.83**

(21) Anmeldenummer: **81102651.7**

(22) Anmeldetag: **08.04.81**

(51) Int. Cl.³: **C 07 C 47/21**, C 07 C 45/67, C 07 C 45/68, C 07 C 29/17, C 07 C 33/02, C 07 C 29/14, A 61 K 7/46, A 23 L 1/226, C 11 B 9/00

(54) **2.4-Dialkyl-2.6-heptadienale und -heptadienole, Verfahren zu deren Herstellung und ihre Anwendung als Geruchs- und Geschmacksstoffe.**

(30) Priorität: **09.04.80 DE 3013672**

(43) Veröffentlichungstag der Anmeldung: **14.10.81 Patentblatt 81/41**

(45) Bekanntmachung des Hinweises auf die Patenterteilung: **16.11.83 Patentblatt 83/46**

(84) Benannte Vertragsstaaten: **BE CH DE FR GB IT LI NL**

(56) Entgegenhaltungen:
**CH A 479 515**
**DE A 2 439 140**
**US A 4 010 207**

**H. JANISTYN, Handbuch d. Kosmetika d. Riechstoffe Bd. 2, Heidelberg 1969, Kosmetika, Riechstoffe und Lebensmittelzusatzstoffe, Stuttgart 1978; Perfumes, Cosmetics and Soaps, Vol. 1, London 1974**

**Die Akte enthält technische Angaben, die nach dem Eingang der Anmeldung eingereicht wurden und die nicht in dieser Patentschrift enthalten sind.**

(73) Patentinhaber: **Consortium für elektrochemische Industrie GmbH**
**Zielstattstrasse 20**
**D-8000 München 70 (DE)**

(72) Erfinder: **Gebauer, Helmut, Dr., Dipl.-Chem.**
**Schaffhauser Strasse 18/VII**
**D-8000 München 71 (DE)**
Erfinder: **Hafner, Walter, Dr., Dipl.-Chem.**
**Sommerfeld 15**
**D-8024 Furth (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 037 584 B1

Jouve, 18, rue St-Denis, 75001 Paris, France

## 2.4-Dialkyl-2.6-heptadienale und -heptadienole, Verfahren zu deren Herstellung und ihre Anwendung als Geruchs- und Geschmacksstoffe

Die Erfindung betrifft 2.4-Dialkyl-2.6-Heptadienale, durch Anlagerung von Wasserstoff an mindestens eine Doppelbindung zu erhaltende Derivate, Verfahren zu deren Herstellung und ihre Anwendung als Geruchs- und Geschmacksstoffe.

Isoprenoidartige 2.6-Diene wurden bisher durch Isolierung und ggf. Umwandlung von Naturstoffen gewonnen. Aldehyde dieser Struktur, wie z. B. Citral, gelangen zu vielfältigen Anwendungen als Riechstoffkomponenten und Lebensmittelzusätze, sowie als Zwischenprodukte für Insektizide und Pharmazeutika.

So werden im « Handbuch der Kosmetika und Riechstoffe » Band II, Seiten 143 und 144 eine Reihe von Riechstoffen beschrieben, die sich von dem bekannten Citral, einem 2.6-Octadienal ableiten.

In « Kosmetika, Riechstoffe und Lebensmittelzusatzstoffe » H. Aegi et al werden ferner aliphatische Riechstoffe mit monoterpenoider Struktur beschrieben. Es handelt sich um tert. Alkohole sowie um einige Homologe des Octylalkohols. Daneben wird gemäß « Perfumes, Cosmetics and Soaps » Dimethyloctanol als Riechstoff genannt.

Vollsynthetische Produkte erforderten bisher zumeist vielstufige Verfahren, wobei kostspielige Reaktionsschritte, wie die Wittig-Reaktion und der Einsatz metallorganischer Verbindungen oft unumgänglich waren.

So wird gemäß CH 479 515 ein Herstellungsverfahren für ungesättigte Aldehyde durch Kondensieren eines Vinylethers mit Allylalkoholen in Gegenwart von Metallsalzen, die Olefinkomplexe bilden, beschrieben.

Ferner ist gemäß DE-OS 24 39 140 ein Verfahren zur Herstellung ungesättigter Aldehyde durch Wärmebehandlung der entsprechenden Allylacetale offenbart. Es finden sich allgemein gehaltene Ausführungen darüber, daß die Verfahrensprodukte als Duftstoffe, Arzneimittel, Pestizide oder als Zwischenprodukte für derartige Wirkstoffe Verwendung finden könnten.

Aufgabe der Erfindung war es nun, für vorstehenden Verwendungszweck geeignete Verbindungen aufzufinden, sowie einfache, vollsynthetische Verfahren zu deren Herstellung anzugeben.

Dieser Aufgabenstellung gerecht werden 2.4-Dimethyl-2.6-heptadienal, 2.4-Diethyl-2.6-heptadienal, 2.4-Di-n-propyl-2.6-heptadienal, 2.4-Di-iso-propyl-2.6-heptadienal, 2.4-Dimethyl-2.6-heptadienol, 2.4-Diethyl-heptanol.

Das Verfahren zur Herstellung von 2.4-Dimethyl-2.6-heptadienal, 2.4-Diethyl-2.6-heptadienal, 2.4-Di-n-propyl-2.6-heptadienal und 2.4-Di-iso-propyl-2.6-heptadienal ist dadurch gekennzeichnet, daß Verbindungen der allgemeinen Formel

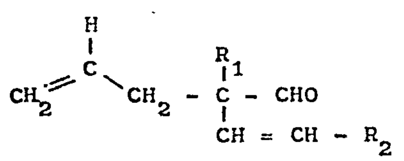

wobei $R_1$ und $R_2$ gleiche aliphatische Reste sind mit der Bedeutung Methyl, Ethyl, Propyl und Iso-propyl einer Temperaturbehandlung von 80 bis 300 °C, vorzugsweise 150 bis 250 °C, unterworfen werden.

Überraschenderweise gehen diese Verbindungen trotz mannigfaltiger sterischer Hinderung unter diesen Bedingungen Umlagerungsreaktionen zu den erfindungsgemäßen Verbindungen ein.

Um die Bildung von Vernetzungs- und Oxidationsprodukten zu vermeiden ist es oftmals zweckmäßig, die Temperaturbehandlung unter Luftausschluß durchzuführen. Geeignete Maßnahmen hierfür sind Arbeiten im geschlossenen System oder Begasen des Reaktionsgemisches mit Inertgasen, wie Stickstoff, Argon u. a.

Erfindungsgemäß wird die Temperaturbehandlung bevorzugt bei Drücken von etwa 1 bar ausgeführt. Es kann jedoch, falls erwünscht, auch bei höheren oder niedrigeren Drücken umgesetzt werden. Für die Synthese erfindungsgemäßer Verbindungen mit sperrigen Resten $R_1$ und $R_2$ sind, zur Vermeidung allzu langer Reaktionszeiten, zuweilen die höheren anspruchsgemäßen Temperaturbedingungen und Drücke bis zu 10 bar zu empfehlen, während sonst vorzugsweise bei 150 bis 250 °C gearbeitet wird.

Die Ausgangsverbindungen können in an sich bekannter Weise, durch Umsetzen aliphatischer Aldehyde mit mindestens 3 Kohlenstoffatomen mit Allyl-Halogeniden in einer phasentransferkatalysierten Reaktion, gewonnen werden (vgl. hierzu die Japanische Offenlegungsschrift 53-141 210).

Alternativ lassen sich auch α-β-ungesättigte Aldehyde, wie sie beispielsweise durch Aldolkondensationen zugänglich sind, als Primärprodukte einsetzen, aus denen ebenfalls, über eine α-Substitutionsreaktion mit Allyl-Halogeniden, die entsprechenden Ausgangsverbindungen gewonnen werden können (vgl. hierzu US-PS 4 010 207).

Erfindungsgemäß fallen im allgemeinen Cis-Trans-Isomerengemische an (E- und Z-Form).

E- und Z-Form können beispielsweise durch H-NMR-Spektroskopie qualitativ unterschieden und quantitativ bestimmt werden. Der Anteil an jeweiligem Isomer am Isomerengemisch wird vom Platzbedarf der Substituenten $R_1$ und $R_2$ mitbestimmt : bei sperrigen Resten wird das Gleichgewicht im allgemeinen

zugunsten der Z-Form verschoben.

Erfindungsgemäß kommen die Isomerengemische zum Einsatz. Die Gemische können jedoch auch, falls erwünscht, durch bekannte Methoden, wie beispielsweise Chromatographie in ihre Einzelkomponenten getrennt werden. Gegenstand der Erfindung sind sowohl die Isomerengemische wie die Einzelkomponenten.

Weiterhin betrifft die Erfindung solche Verbindungen, die sich durch Anlagerung von Wasserstoff an mindestens eine Doppelbindung herleiten, wobei es sich um eine Kohlenstoffdoppelbindung und/oder die Carbonylfunktion handeln kann.

Die Anlagerung von Wasserstoff an mindestens eine Doppelbindung der erfindungsgemäßen 2.4-Dialkyl-2.6-Heptadienale kann in an sich bekannter Weise erfolgen. Zu unterscheiden sind selektive und erschöpfende Hydrierungsmaßnahmen :

Beispiele für solche Hydrierungsagentien, die selektiv die Carbonylfunktion hydrieren, sind Natriumboranat, Lithiumalanat und andere ;

Totalhydrierungen lassen sich beispielsweise mit molekularem Wasserstoff in Gegenwart von Raney-Nickel erzielen.

Die erfindungsgemäßen Verbindungen sind aus großtechnisch zugänglichen Basischemikalien — aliphatischer Aldehyd und Allyl-Halogeniden — durch einfache chemische Operationen zugänglich. Für solche isoprenoidartige Aldehyde bzw. deren hydrierte Derivate eröffnet sich ein weites Anwendungsfeld : so sind beispielsweise bei ihrer Verwendung als Komponenten für Geruchs- oder Lebensmittelzusatzstoffe citrus-, holz- oder blumenartige Duft- und Geschmacksnoten erzielbar. Sie sind ferner als Zwischenprodukte für Insektizide, Lockstoffe und Juvenilhormone von besonderem Interesse. Darüberhinaus dienen sie als Monomere zur Herstellung wertvoller Polymerisate.

Die Erfindung wird nun anhand von Beispielen näher erläutert :

## Beispiel 1

Herstellung von 2.4-Di-methyl-2.6-Heptadienal

a) 2-Allyl-2-methyl-3-pentenal

In einem 1 l-Vierhalskolben mit Rührer, Thermometer, Tropftrichter und Rückflußkühler werden 160 g 5 %-ige Natronlauge, 50 ml Wasser und 10 g Tetrabutylammoniumjodid vorgelegt und auf 60 °C erwärmt. Unter kräftigem Rühren wird nun ein Gemisch aus 61,5 ml (0,75 Mol) Allylchlorid und 72 ml (1 Mol) Propionaldehyd so schnell zugetropft, daß eine Temperatur von 65 bis 70 °C gehalten wird (ca. 1 Stunde). Das Reaktionsgemisch wird noch 2 Stunden auf 60 °C gehalten, anschließend mit 100 ml Wasser versetzt, kurz gerührt, die Phasen getrennt, die wäßrige Phase zweimal mit je 100 ml Toluol extrahiert und die vereinigten organischen Phasen schließlich nach Abziehen des Lösungsmittels über eine 30 cm-Vigreuxkolonne fraktioniert destilliert.

Es werden 31,7 g (46 % der Theorie, bezogen auf eingesetzten Aldehyd) 2-Allyl-2-methyl-3-pentenal erhalten. $Kp_{12}$ 57 bis 60 °C.

b) 2.4-Di-methyl-2.6-Heptadienal

In einem 100 ml-Kolben mit Rückflußkühler werden 61 g 2-Allyl-2-methyl-3-pentenal unter langsamem Durchleiten von gasförmigem Stickstoff bis zur vollständigen Umlagerung (GC-Kontrolle) auf 160 °C erhitzt. Nach ca. 14 Stunden erbringt die anschließende franktionierte Destillation 39,8 g (78 % der Theorie) ein cis-Trans-Isomeren-Gemisch von 2.4-Di-methyl-2.6-Heptadienal.

Farbloses Öl ; $Kp_{11}$ 72 bis 75 °C ; 92 % E- und 8 % Z-Form.

## Beispiel 2

Herstellung von 2.4-Di-ethyl-2.6-Heptadienal

a) 2-Allyl-2-ethyl-3-hexenal

In einer 50 l-Glasapparatur mit Rührer, Rückflußkühler und Tropftrichter werden 6 kg 50 %-ige Natronlauge, 2,5 l Wasser, 8 l Toluol und 200 g Tetrabutylammoniumjodid vorgelegt. Innerhalb von zwei Stunden läßt man unter Rühren ein Gemisch aus 4,6 l (50 Mol) Butyraldehyd und 3 l (37,5 Mol) Allylchlorid zufließen. Die Kesseltemperatur wird bei 62 bis 68 °C gehalten. Nach 7 Stunden Nachreaktion bei der gleichen Temperatur wird die wäßrige Phase abgetrennt. Die organische Phase wird zweimal mit je 3 l Wasser gewaschen und schließlich über eine 140 cm Füllkörperkolonne destilliert.

Ausbeute 2,82 kg (68 % der Theorie, bezogen auf den eingesetzten Aldehyd)

Farbloses Öl ; $Kp_{12}$ 82 bis 83 °C.

b) 2.4-Di-ethyl-2.6-Heptadienal

128 g Aldehyd aus Ansatz a) werden unter Stickstoff 10 Stunden auf 180 °C erhitzt. Die GC-Kontrolle ergibt nahezu quantitativen Umsatz. Durch anschließende Destillation über eine 30 cm-Vigreux-Kolonne werden 110 g (86 % der Theorie) eines Isomerengemisches aus 96 % E- und 4 % Z-Form gewonnen.

Farbloses Öl ; $Kp_{12}$ 92 bis 93 °C.

Beispiel 3

Herstellung von 2.4-Di-n-propyl-2.6-Heptadienal

a) 2-Allyl-2-n-propyl-3-heptenal

In einem 2 l-Vierhalskolben mit Rührer, Thermometer, Tropftrichter und Rückflußkühler werden 400 g 50 %-ige Natronlauge, 125 ml Wasser, 400 ml Toluol und 25 g Tetrabutylammoniumbromid vorgelegt. Unter Rühren wird, bei einer Temperatur zwischen 70 und 80 °C, eine Mischung aus 280 ml (2,5 Mol) n-Valeraldehyd und 155 ml (1,9 Mol) Allylchlorid innerhalb 35 Minuten zugetropft. Nach 6 Stunden Nachreaktion bei 80 °C werden die Phasen getrennt, die organische Phase mit 100 ml Wasser gewaschen und über eine 30 cm-Vigreux-Kolonne destilliert.

Das Reaktionsprodukt fällt als farbloses Öl vom $Kp_{12}$ 109 bis 111 °C an.

Ausbeute 165 g (68 % der Theorie, bezogen auf den eingesetzten Aldehyd).

b) 2.4-Di-n-propyl-2.6-Heptadienal

80 g Aldehyd aus a) werden im Glaskolben unter Rühren und Luftausschluß 14 Stunden bis zum nahezu quantitativen Umsatz (GC-Kontrolle) auf 185 °C erhitzt. In der Hauptfraktion destillierten anschließend 68,5 g (86 % der Theorie) Produkt als Gemisch aus 80 % E- und 20 % Z-Form über.

Farbloses Öl ; $Kp_9$ 114 °C.

Beispiel 4

Herstellung von 2.4-Di-i-propyl-2.6-Heptadienal

a) 2-Allyl-2-i-propyl-5-methyl-3-hexenal

In einem 2 l-Vierhalskolben mit Rührer, Thermometer, Tropftrichter und Rückflußkühler werden 400 g 50 %-ige Natronlauge, 125 ml Wasser, 400 ml Toluol und 25 g Tetrabutylammoniumbromid vorgelegt. Unter Rühren bei einer Temperatur von 65 °C wird ein Gemisch aus 376 ml (3,5 Mol) Isovaleraldehyd und 155 ml (1,9 Mol) Allylchlorid innerhalb von 45 Minuten eindosiert. Nach 4 Stunden Nachreaktion bei der gleichen Temperatur werden die Phasen getrennt, die wäßrige Phase mit 100 ml Toluol extrahiert, die vereinigten organischen Phasen mit wasserfreiem Natriumsulfat getrocknet und über eine 30 cm-Vigreux-Kolonne fraktioniert destilliert.

Ausbeute 224 g (66 % der Theorie, bezogen auf den eingesetzten Aldehyd).

Farbloses Öl ; $Kp_{0,01}$ 49 °C.

b) 2.4 Di-i-propyl-2.6-Heptadienal

100 g Aldehyd aus a) werden in einem Glaskolben unter Rühren und Luftausschluß auf 180 °C erhitzt. Nach 20 Stunden zeigt das GC-Spektrum vollständigen Umsatz an. Die folgende Destillation erbringt 84 g (84 % der Theorie) an Zielprodukt (Isomerenverhältnis 1 : 1).

Farbloses Öl ; $Kp_{12}$ 104 bis 106 °C.

Beispiel 5

2.4-Di-ethylheptanol

In einem 0,5 l-Schüttelautoklaven mit Glaseinsatz werden 33,2 g (0,2 Mol) 2.4-Di-ethyl-2.6-Heptadienal, 70 ml Methanol und 3 g frisch hergestelltes Raney-Nickel gegeben. Die Hydrierung nimmt (bis zur Druckkonstanz) ca. 165 Minuten in Anspruch. Anschließend wird vom Katalysator abfiltriert und nach Abziehen des Lösungsmittels im Wasserstrahlvakuum destilliert.

Die Ausbeute beträgt 28 g, entsprechend 81,4 % der Theorie.

$Kp_{12}$ 109 °C, farbloses Öl, fruchtartiger Geruch.

Beispiel 6

2.4-Di-methyl-2.6-Heptadienol

In einem 250 ml-Zweihalskolben mit Tropftrichter und Rückflußkühler werden 1,1 g (0,029 Mol) $LiAlH_4$ in 100 ml absolutem Äther vorgelegt. Unter Rühren wird nun eine Lösung von 13,8 g (0,1 Mol) 2.4-Di-methyl-2.6-Heptadienal in 40 ml absolutem Äther so schnell zugetropft, daß das Gemisch mäßig siedet. Nach einer Stunde Nachreaktion und Rückfluß wird mit Eiswasser hydrolysiert, mit verdünnter Schwefelsäure schwach angesäuert, die Phasen getrennt und die organische Phase nach Trocknen mit $Na_2SO_4$ im Wasserstrahlvakuum destilliert.

Die Ausbeute beträgt 11,1 g, entsprechend 79,3 % der Theorie.

$Kp_{12}$ 90 °C, farbloses Öl, zitronenartiger, fruchtiger Geruch.

## Ansprüche

1. 2.4-Dimethyl-2.6-heptadienal, 2.4-Diethyl-2.6-heptadienal, 2.4-Di-n-propyl-2.6-heptadienal, 2.4-Di-iso-propyl-2.6-heptadienal, 2.4-Dimethyl-2.6-heptadienol, 2.4-Diethyl-heptanol.

2. Verfahren zur Herstellung von 2.4-Dimethyl-2.6-heptadienal, 2.4-Diethyl-2.6-heptadienal, 2.4-Di-n-propyl-2.6-heptadienal und 2.4-Di-iso-propyl-2.6-heptadienal, dadurch gekennzeichnet, daß Verbindungen der Formel

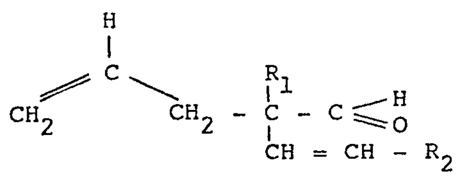

wobei $R_1$ und $R_2$ gleiche aliphatische Reste sind mit der Bedeutung Methyl, Ethyl, Propyl und Iso-propyl einer Temperaturbehandlung von 80 bis 300 °C, vorzugsweise 150 bis 250 °C unterworfen werden.

3. Verfahren zur Herstellung von 2.4-Dimethyl-2.6-heptadienol, dadurch gekennzeichnet, daß 2.4-Dimethyl-2.6-heptadienal mit $NaBH_4$ oder $LiAlH_4$ behandelt wird.

4. Verfahren zur Herstellung von 2.4-Diethyl-heptanol, dadurch gekennzeichnet, daß 2.4-Diethyl-2.6-heptadienal in Gegenwart von Raney-Nickel mit molekularem Wasserstoff hydriert wird.

5. Verwendung von Verbindungen nach Anspruch 1 als Geruchs- und Geschmacksstoffe.

## Claims

1. 2,4-Dimethyl-2,6-heptadienal, 2,4-diethyl-2,6-heptadienal, 2,4-di-n-propyl-2,6-heptadienal, 2,4-diisopropyl-2,6-heptadienal, 2,4-dimethyl-2,6-heptadienol and 2,4-diethylheptanol.

2. Process for the manufacture of 2,4-dimethyl-2,6-heptadienal, 2,4-diethyl-2,6-heptadienal, 2,4-di-n-propyl-2,6-heptadienal and 2,4-diisopropyl-2,6-heptadienal, characterised in that compounds of the formula

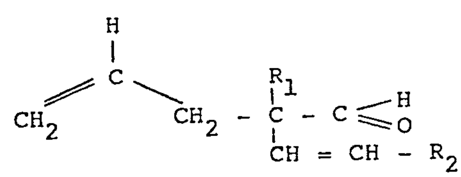

in which $R_1$ and $R_2$ are the same aliphatic radicals and are methyl, ethyl, propyl or isopropyl, are subjected to heat treatment at from 80 to 300 °C, preferably from 150 to 250 °C.

3. Process for the manufacture of 2,4-dimethyl-2,6-heptadienol, characterised in that 2,4-dimethyl-2,6-heptadienal is treated with $NaBH_4$ or $LiAlH_4$.

4. Process for the manufacture of 2,4-diethylheptanol, characterised in that 2,4-diethyl-2,6-heptadienal is hydrogenated with molecular hydrogen in the presence of Raney nickel.

5. Use of compounds according to claim 1 as odoriferous substances and flavouring substances.

## Revendications

1. Diméthyl-2,4 heptadiène-2,6 al, diéthyl-2,4 heptadiène-2,6 al, di-n-propyl-2,4 heptadiène-2,6 al, diisopropyl-2,4 heptadiène-2,6 al, diméthyl-2,4 heptadiène-2,6 ol et diéthyl-2,4 heptanol.

2. Procédé de préparation du diméthyl-2,4 heptadiène-2,6 al, du diéthyl-2,4 heptadiène-2,6 al, du di-

n-propyl-2,4 heptadiène-2,6 al et du diisopropyl-2,4 heptadiène-2,6 al, procédé caractérisé en ce qu'on soumet des composés répondant à la formule :

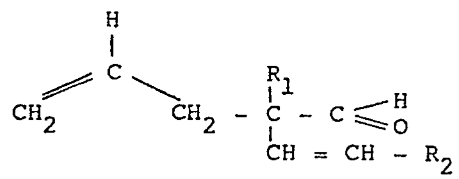

dans laquelle $R_1$ et $R_2$ représentent des radicaux aliphatiques identiques qui sont des radicaux méthyles, éthyles, propyles et isopropyles, à un traitement par la chaleur à une température de 80 à 300 °C, de préférence de 150 à 250 °C.

3. Procédé de préparation du diméthyl-2,4 heptadiène-2,6 ol, procédé caractérisé en ce qu'on traite le diméthyl-2,4 heptadiène-2,6 al par $NaBH_4$ ou $LiAlH_4$.

4. Procédé de préparation du diéthyl-2,4 heptanol, procédé caractérisé en ce qu'on hydrogène le diéthyl-2,4 heptadiène-2,6 al par de l'hydrogène moléculaire en présence de nickel de Raney.

5. Application de composés selon la revendication 1 comme substances odorifiques et saporifiques.